(19) Europäisches Patentamt
European Patent Office
Office européen des brevets

(11) EP 0 628 539 B1

(12) **EUROPEAN PATENT SPECIFICATION**

(45) Date of publication and mention of the grant of the patent:
**11.08.1999 Bulletin 1999/32**

(51) Int Cl.[6]: **C07C 229/24**, C07C 309/14, C07C 59/305, C07C 309/17, C07C 323/52, C02F 5/10, C02F 5/12

(21) Application number: **94303602.0**

(22) Date of filing: **20.05.1994**

(54) **Polyepoxysuccinic acid derivatives and their use for the control of scale formation and corrosion in aqueous systems**

Polyepoxybernsteinsäurederivate und ihre Verwendung zur Kesselsteinverhütung und Korrosionskontrolle in wässrigen Systemen

Dérivés de l'acide polyepoxysuccinique et leur utilisation pour l'inhibition de la formation de tartre et pour le contrôle de la corrosion dans des systèmes aqueux

(84) Designated Contracting States:
**AT BE DE ES FR GB IE IT NL PT**

(30) Priority: **09.06.1993 US 74254**
**13.08.1993 US 106452**

(43) Date of publication of application:
**14.12.1994 Bulletin 1994/50**

(73) Proprietor: **BetzDearborn Europe, Inc.**
**Trevose PA 19053-6783 (US)**

(72) Inventors:
- **Carey, William Sean**
  **Ridley Park, PA 19078 (US)**
- **Ehrhardt, William Christopher**
  **Hamilton NJ 08690 (US)**
- **Perez, Libardo Aquiles**
  **Morrisville PA 19067 (US)**
- **Solov, Andrew**
  **Holland PA 18966 (US)**
- **Freese, Donald Todd**
  **Glenside PA 19038 (US)**

(74) Representative: **W.P. Thompson & Co.**
**Coopers Building,**
**Church Street**
**Liverpool L1 3AB (GB)**

(56) References cited:
**EP-A- 0 460 797**
**DE-A- 4 212 755**
**DE-A- 4 224 607**
**US-A- 3 776 850**
**US-A- 4 152 515**
**US-A- 4 654 159**
**US-A- 4 846 650**

- **PATENT ABSTRACTS OF JAPAN vol. 16, no. 466 (C-0989) 28 September 1992 & JP-A-04 166 298 (KAO) 12 June 1992**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to the treatment of water to inhibit the formation of scale. More particularly, the present invention relates to the use of a modified polyepoxysuccinic acid to inhibit scale formation and prevent corrosion of ferrous-based metals in contact with aqueous systems.

In industrial cooling systems, water such as from rivers, lakes, ponds, etc., is employed as the cooling media for heat exchangers. Such natural waters contain large amounts of suspended materials such as silt, clay, and organic wastes. The cooling water from heat exchangers is typically passed through a cooling tower, spray pond or evaporative system prior to discharge or reuse. In these systems, the cooling effect is achieved by evaporating a portion of the water passing through the system. Because of the evaporation which takes place during cooling, suspended materials in the water become concentrated. Fouling materials from the feedwater or as a result of evaporative concentration can settle in locations of low flow rate and cause corrosion and inefficient heat transfer. Agglomerating agents such as polyacrylamides and polyacrylates have been used to agglomerate fine particles of mud and silt into a loose floc for removal. However, these flocs tend to settle in cooling tower basins and frequent cleaning is necessary to remove the settled flocs from the tower basins.

The water employed in industrial cooling water systems also often contains dissolved salts of calcium and magnesium, etc., which can lead to scale and sludge deposits. One of the most common scale deposits in cooling water systems is calcium carbonate. It normally results from the breakdown of calcium bicarbonate, a naturally occurring soluble salt. Calcium carbonate has a relatively low solubility and its solubility decreases with increasing temperature and pH. Thus, the rate of calcium carbonate deposition increases with increasing pH and temperature.

Deposit control agents such as phosphates, phosphonates and polyacrylates are often used to inhibit calcium carbonate scale formation in industrial cooling water systems. The use of polyacrylates alone is not effective at high calcium concentrations because undesirable polyacrylate-calcium precipitates are formed reducing efficiency.

Although phosphonates are very effective at controlling calcium carbonate scale formation, and certain phosphonates exhibit excellent calcium tolerance, i.e. the ability to inhibit calcium carbonate scale in water having a propensity toward scale deposition, they can produce insoluble phosphonate-calcium complexes or calcium phosphate scale upon degradation, especially in waters having high calcium concentrations and pH. Further, current limits on total phosphorus discharge (as P) due to environmental concerns limit the acceptability of the use of phosphonates for water treatment.

In cooling systems, corrosion causes two basic problems. The first and most obvious is the failure of equipment, resulting in replacement costs and plant downtime. Also, decreased plant efficiency occurs due to the loss of heat transfer. The accumulation of corrosion products causes heat exchanger fouling, resulting in the loss of heat transfer.

Ferrous-based metals, e.g., iron metal and metal alloys containing iron (mild steel), are routinely used in the construction of cooling systems due to their low cost and availability. As the system water passes over or through these ferrous-based metal containing devices, they are subjected to corrosion processes. Corrosion inhibitors are generally added as part of a water treatment program in cooling systems to prevent and inhibit the corrosion of ferrous-based metal containing devices.

Chromates, molybdates, zinc, phosphates or polyphosphates, and phosphonates have been used to inhibit the corrosion of ferrous-based metals in contact with the system water of cooling systems. Each treatment, however, presents certain drawbacks. Chromate is highly toxic and presents handling and disposal problems. Phosphates, polyphosphates, and phosphonates contribute to the eutrophication of the receiving water upon discharge, leading to the restriction of their discharge by regulatory bodies. The discharge of cooling tower blowdown containing zinc, a heavy metal, is also regulated due to its aquatic toxicity. Molybdate and tungstate are not effective at low concentrations and generally are combined with conventional inhibitors, such as phosphonates, to be cost effective.

There exists a need, therefore, for a more environmentally acceptable corrosion inhibitor of ferrous-based metals in contact with aqueous systems. In particular, there is a need for a non-phosphorous containing organic corrosion inhibitor.

Preventing the corrosion and scaling of industrial heat transfer equipment is essential to the efficient and economical operation of a cooling water system. Excessive scaling and/or corrosion of metallic surfaces can cause the premature failure of process equipment, necessitating downtime for the replacement or repair of the equipment. Additionally, the buildup of scale and corrosion products on the heat transfer surface reduces efficiency, thereby limiting production or requiring downtime for cleaning.

Scale can also cause rapid localized corrosion and subsequent penetration of metallic surfaces through the formation of differential oxygen concentration cells. The localized corrosion resulting from differential oxygen cells originating from deposits is commonly referred to as "underdeposit corrosion".

EP-A- 0 460 797 discloses a method of treating water using a polyepoxysuccinic acid (PESA), unsubstituted or alkyl substituted, or salts thereof as a scale inhibitor.

JP 4,166,298 also discloses the use of PESA or salts thereof for a similar purpose. US 4, 846, 650 discloses a method of retarding formation of dental calculus (tartar, which is calcium phosphate in a hydroxyapatite lattice) using PESA or salts thereof.

US 4, 152, 515 discloses monomeric β-alkoxy-α-hydroxysuccinic acids and β-alkylthio-α-hydroxysuccinic acids and salts thereof for use as precipitant builder compounds in detergent compositions.

The present invention provides an effective method and novel compounds for inhibiting scale formation as well as inhibiting and controlling corrosion of metals, particularly ferrous-based metals in contact with aqueous systems. The present invention is effective at conditions of high pH, high calcium concentration and high M-alkalinity where conventional treatments lose efficacy. The treatment of the present invention also controls calcium scale formation without forming undesirable inhibitor-calcium precipitates. Also, the method of the present invention does not require phosphorus, thereby reducing or eliminating the undesirable discharge of phosphorus-containing compounds. The method of the present invention allows industrial cooling water systems to operate at higher cycles of concentration, acid feed for pH control can be reduced or eliminated, and phosphorus limited systems can be treated effectively. In addition to treating waters having high calcium levels, the present invention is also effective at treating waters having low levels of calcium.

The present invention provides a novel modified poly(epoxysuccinic acid) of the general formula:

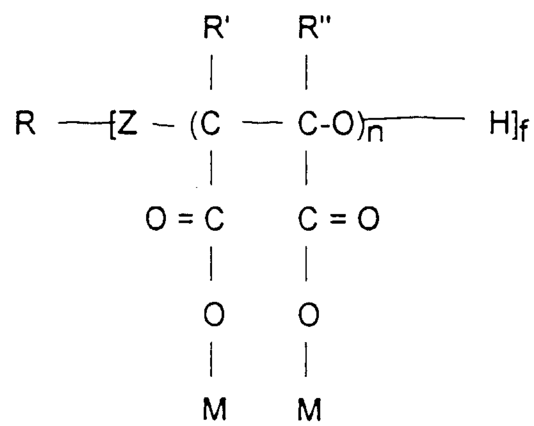

wherein R, when present, is a substituted or non-substituted alkyl or aryl moiety preferably having a carbon chain up to the length where solubility in an aqueous solution is lost, or a repeat unit obtained after polymerization of an ethylenically unsaturated compound; R' and R" each independently are hydrogen, $C_{1-4}$ alkyl or $C_{1-4}$ substituted alkyl; Z is O, S, NH, or NR, where R is as described above; n is a positive integer greater than 1; f is a positive integer; and each M independantly is hydrogen, a water soluble cation (e.g.,$NH_4^+$, alkali metal), or a non-substituted lower alkyl group having from 1 to 3 carbon atoms. (When R is not present, Z may be $MO_3S$, where M is as described above).

In a preferred embodiment of the invention, R is a $C_{1-20}$ alkyl or substituted alkyl moiety, or an aryl or substituted aryl moiety containing 4-9 carbon atoms, R' and R" are hydrogen, Z is NH, n is greater than 1, f is 1-2, and M is $Na^+$.

In a particularly preferred embodiment of the invention, R is a $—CH_2C_6H_4CH_2—$ moiety, R' and R" are hydrogen, Z is NH, n is greater than 1, f is 2, and M is $Na^+$. In another particularly preferred embodiment of the invention, R is $HOCH_2(CHOH)_3C(CO_2H)$-, R' and R" are hydrogen, Z is -0-, n is greater than 1, f is is and M is $Na^+$.

The present invention also provides methods for controlling the formation and deposition of scale forming salts in an aqueous system, and for controlling the corrosion of metals in contact with an aqueous system, comprising introducing the above compounds into the aqueous system.

In the present invention, the modified poly[epoxysuccinic acids] are added to the aqueous system at substoichiometric levels to inhibit scale formation. The method of the present invention provides effective calcium carbonate deposition inhibition in waters having relatively high Langelier saturation indexes. The method of the present invention provides such control at relatively low active treatment levels without the use of phosphates or phosphonates.

The compositions of the present invention should be added to the aqueous system for which corrosion inhibition activity of the ferrous-based metal parts in contact with an aqueous system is desired, in an amount effective for the purpose. This amount will vary depending upon the particular system for which treatment is desired and will be influenced by factors such as the area subject to corrosion, pH, temperature, water quantity and respective concentrations in the water of corrosive species. For the most part, the present invention will be effective when used at levels of from about 25 parts per billion to 500 parts per million of water, and preferably from about 50 parts per billion to 100 parts per million of water contained in the aqueous system to be treated. The present invention may be added directly

to the desired water system in a fixed quantity and in a state of an aqueous solution, continuously or intermittently.

DESCRIPTION OF THE PREFERRED EMBODIMENTS

The present invention pertains to a novel method of inhibiting the formation of scale such as calcium scale from aqueous systems, e.g., cooling water, steam generating, gas scrubbing and pulp and papermaking systems. The present invention also pertains to a novel method of inhibiting and controlling corrosion of ferrous-based metals in contact with such aqueous systems. Specifically, the method of the present invention comprises adding to an aqueous system a modified poly[epoxysuccinic acid] of the general formula:

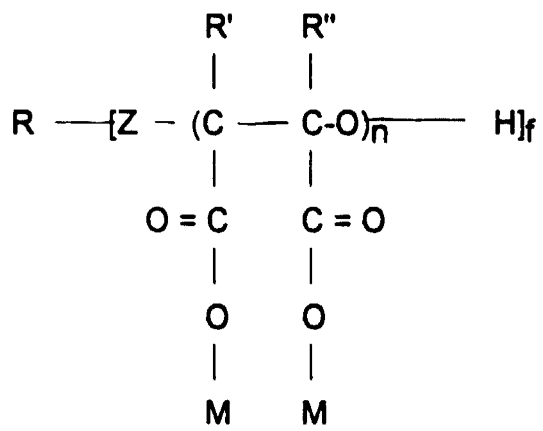

Formula I

wherein R is a substituted or non-substituted alkyl or aryl moiety having a carbon chain up to the length where solubility in an aqueous solution is lost, or a repeat unit obtained after polymerization of an ethylenically unsaturated compound; R' and R" are hydrogen, $C_{1-4}$ alkyl or $C_{1-4}$ substituted alkyl; Z is O, S, NH, or NR, where R is as described above; n is a positive integer greater than 1; f is a positive integer; and each M independantly is hydrogen, a water soluble cation (e.g.,$NH_4^+$, alkali metal), or a non-substituted lower alkyl group having from 1 to 3 carbon atoms.

The compounds of the present invention can be prepared by incorporating reagents containing an α - hydroxycarboxylic acid (α HCA) functionality into a poly[epoxysuccinic acid] (PESA) polymer matrix. The α HCA compounds can be obtained by the ring opening reaction of a suitable reagent $(R-[-Z-H]_f$ with a salt or ester of epoxysuccinic acid (ESA). The α HCA compound can be synthesized prior to the incorporation reaction (Scheme A) or be generated in situ by conducting the polymerization of ESA in the presence of a suitable ring-operating reagent (Scheme B).

Scheme A

Step 1:

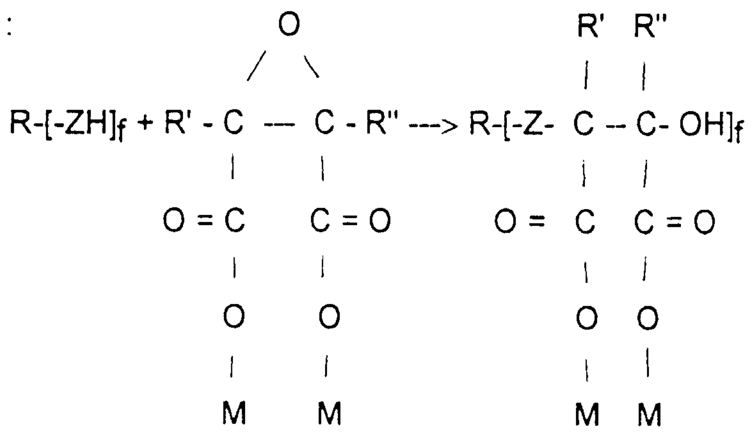

ESA HCA

Step 2:

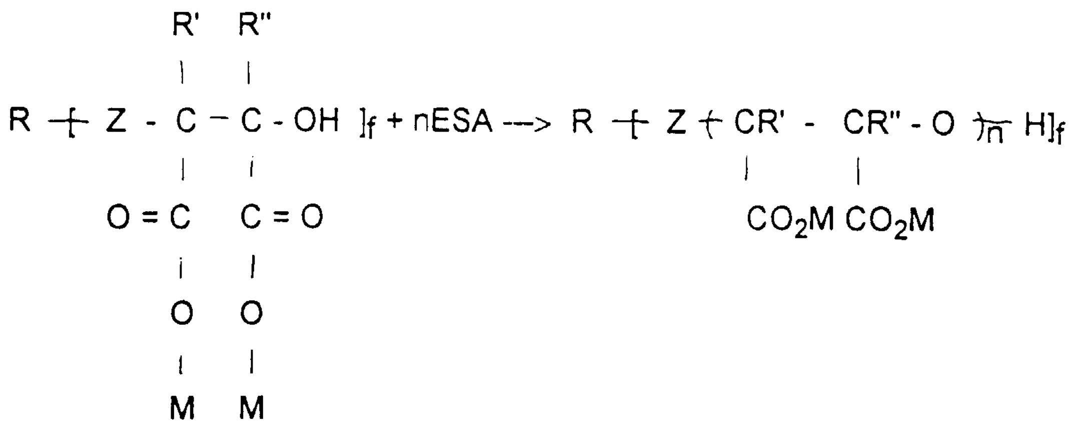

Scheme B

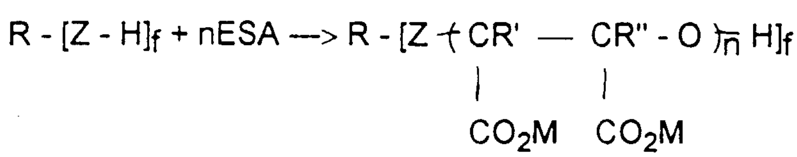

For a general review of ring-opening reactions of epoxides to prepare αHCA compounds, see March, "Advanced Organic Chemistry-Reactions, Mechanisms, and Structures", 2nd Edition, Chapter 10, McGraw-Hill, New York, 1977.

Methods for conducting the polymerization of ESA, Scheme A-Step 2 and Scheme B, are described by Pearson et al., U.S. Patent 3,776,850 and Bush et al., U.S. Patent 4,654, 159.

The reaction can be performed neat, or in aqueous or non-aqueous solvents. If the resulting product is non-aqueous it should be modified by traditional techniques known to those skilled in the art to yield a water soluble product (e.g., hydrolysis of ester derivatives).

In a preferred embodiment of the invention, aqueous solutions of the compounds of the present invention are prepared by reacting an amine with an aqueous solution of disodium epoxysuccinate (ESA · $Na_2$) in the presence of calcium hydroxide. The reaction is typically conducted under atmospheric conditions at about 30°C - 100°C, preferably from 80° C to 100°C. The molar ratio of the ring opening reagent $R[Z - H]_f$ to ESA · $Na_2$, relative to functionality (f) may fall within the range of about 1:2 to 1:1000, with a range of 1:5 to 1:100 being preferred. The molar ratio of calcium hydroxide to ESA $Na_2$ or ESA $Na_2$ + αHCA may fall within the range of 1:20 to 1:3, with a ratio of 1:10 being preferred.

It will be appreciated that certain by-products (e.g., disodium tartrate, PESA, and αHCA compounds) may be produced along with the compounds of the present invention in the course of the above reaction schemes. The desired reaction products can be readily recovered from the reaction product by known methods; however, it is feasible and economical to employ the compounds of the present invention as produced without separation or purification.

The treatment levels of compound added to an aqueous system can range from 25 parts per billion to 500 parts per million of water, and preferably from 50 parts per billion to 100 parts per million of water contained in the aqueous system to be treated. The concentration of compound necessary to provide effective calcium control will, of course, vary from system to system. The treatment level will vary, in part, with changes in temperatures and pH. However, in all cases, the concentration of modified polyepoxysuccinic acid added to an aqueous water system in accordance with the present invention is at substoichiometric concentrations. That is, the concentration of modified polyepoxysuccinic acid added is much lower than the concentration of the scale forming material in the system to be treated.

The concentration of compound necessary to provide effective corrosion inhibition will also vary from system to system. The treatment level will vary, in part, with changes in temperature and pH, water quantity and respective concentrations in the water of corrosive species.

The compounds may be added directly into the desired water system in a fixed quantity and in a state of an aqueous solution, continuously or intermittently. As noted, the compounds of the present invention are expected to exhibit scale inhibition, e.g., calcium carbonate, barium sulfate, calcium oxalate, calcium sulfate and silica/silicate activity, as well as corrosion inhibition activity. In addition, the compounds of the present invention may also be used with topping agent components in order to enhance the scale controlling and corrosion inhibition properties thereof. Such topping components are readily known to those skilled in the art. Details of such compounds are disclosed by Chen, U.S. Patent 4,659,481. It is expected that the compounds of the present invention may be used in conjunction with the polymers and topping components of Chen '481 to provide treatment programs which effectively inhibit corrosion and scale deposition in water systems.

Suitable topping agents include polyacrylates, phosphoric acid and water soluble salts thereof, phosphonic acids and water soluble salts thereof, polyvalent metal salts, azole compounds, molybdate and tungstate compounds and mixtures thereof.

A suitable polyacrylate is represented by the following formula:

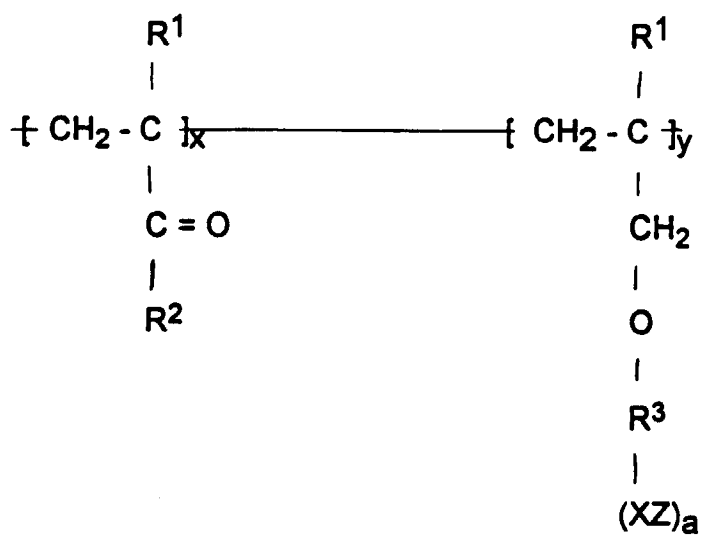

wherein $R^1$ is H or lower alkyl ($C_{1-3}$); $R^2$ is OH, OM or $NH_2$; M is a water soluble cation; $R^3$ is a hydroxy substituted alkyl or alkylene radical having from 1 to 6 carbon atoms or a non-substituted alkyl or alkylene radical having from 1

to 6 carbon atoms; X, when present, is an anionic radical selected from $SO_3$, $PO_3$, $PO_4$ and $CO_2$; Z, when present, is H or any water soluble cation or cations which together counterbalance the valence of the anionic radical; a is 0 or 1, the molar ratio of x:y of the polymer being between 30:1 and 1:20.

The phosphoric acid may be orthophosphoric acid or pyrophosphoric acid or a water soluble salt thereof. The phosphonic acid may be 1-hydroxyethane-1, 1-diphosphonic acid, 2-phosphonobutane-1,2,4-tricarboxylic acid or hydroxyphosphonoacetic acid. The polyvalent metals may be $Zn^{2+}$, $Mn^{2+}$, or $Sn^{2+}$. The azole compound may be 1,2,3,tolyltriazole, benzotriazole or butylbenzotriazole. The molybdate compound may be sodium molybdate or potassium molybdate. The tungstate compound may be sodium or potassium tungstate.

The topping agents may be added to the system in an amount of about 0.01 to 500 ppm of said system.

The invention will now be further described with reference to a number of specific examples which are to be regarded solely as illustrative and not as restricting the scope of the present invention.

<u>Example 1</u>

Preparation of aspartic acid, 3-hydroxy, N-[2-ethanesulfonic acid]trisodium salt via Reaction Scheme A, Step 1.

A suitable reaction flask was equipped with a magnetic stirrer, reflux condenser, nitrogen sparge, thermometer, and addition ports. Taurine (99%, 12.64g, 0.1 mole) and 67 ml of deionized water were charged to the flask and sparged with nitrogen. Aqueous sodium hydroxide (50%, 8g, 0.1 mole) was then charged to the flask to yield a clear, colorless liquid followed by ESA · $Na_2$ (90%, 20.54g, 0.105 mole) being charged to the flask. The resulting slurry was heated at 90 ± 2°C for 16.5 hours under a nitrogen atmosphere. The resulting clear solution was isolated and diluted to 130g with deionized water.

The structure of the resulting aspartic acid, 3-hydroxy, N-[2-ethanesulfonic acid]- trisodium salt, sample reference HCA(9), was confirmed by $^{13}C$ NMR spectroscopy. The product yield was estimated to be 93.3 mole % via integration of the methine carbons of the $^{13}C$ NMR spectrum.

<u>Example II</u>

Preparation of poly[oxy(1,2-dicarboxylic acid -1, 2-ethanediyl)] -$\alpha$ hydro-$\omega$- [(ethanesulfonic acid) 2-amino]-sodium salt via Reaction Scheme A, Step 2.

To a reactor setup similar to that described in Example I was charged aqueous aspartic acid, 3-hydroxy, N-[2-ethanesulfonic acid]-trisodium salt (29.2%, 11.07g, 0.01 mole), 48 ml deionized water, and ESA · $Na_2$ (90%, 37.16g, 0.19 mole). The solution was sparged with nitrogen and adjusted to a pH of 10.8 with aqueous sodium hydroxide (50%). Calcium hydroxide (98%, 1.51g, 0.02 mole) slurried in 20 ml of deionized water was then charged to the flask and the mixture was heated to 80 ± 2°C for 15.5 hours. The resulting solution was then filtered, diluted to 130g with deionized water, and collected.

The structure of the product, Sample Reference 20, was verified by $^{13}C$ NMR spectroscopy. Residual 3-hydroxy, N-[2-ethanesulfonic acid]-trisodium salt was also detected. Approximately 23.4 mole % of the ESA · $Na_2$ hydrolyzed to disodium tartrate under these reaction conditions.

<u>Example III</u>

Preparation of poly[oxy(1,2-dicarboxylic acid-1,2-ethanediyl)] $\alpha$-hydro-$\omega$-[(ethanesulfonic acid)2-amino]-sodium salt via Reaction Scheme B.

To a reactor setup similar to that described in Example I was charged ESA · $Na_2$ (90%, 19.56g, 0.1 mole), 27 ml deionized water, and taurine (99%, 0.63g, 0.005 mole). The solution was sparged with nitrogen and adjusted to a pH of 10.1 with aqueous sodium hydroxide (50%). Calcium hydroxide (98%, 0.76g, 0.01 mole) slurried in 10 ml of deionized water was then charged to the flask and the mixture was heated at 80 ± 2°C for 17 hours. The resulting solution was then filtered, diluted to 65g with deionized water, and collected.

The $^{13}C$ NMR of the product, Sample Reference 23, was similar to that of Example II. No residual taurine was detected. Approximately 22.2 mole % of the ESA · $Na_2$ hydrolyzed to the disodium tartrate by-product under these reaction conditions.

Using the above-described preparative techniques, several other modified PESA analogs were prepared. The final products were typically a mixture of the modified PESA analog, residual $\alpha$HCA, and unmodified PESA (collectively considered the "actives" portion in testing), and sodium tartrate by-product. The results of these preparations are set forth in Table I. Several $\alpha$HCA analogs (Formula I, n=1) were also prepared for evaluation. These compounds are also listed in Table I for reference.

## TABLE I

### Modified PESA Synthesis Summary[a]

R-[-Z-(-CR'——— CR"-O-)$_n$-H]$_f$
|      |
$CO_2M$   $CO_2M$

R' = R" = H, M = Na, n > 1

| Sample | Mole Ratio $ESA \cdot Na_2 : R-[-Z-H]_f$ | Composition[b] Wt. % Actives: Wt. % $TA \cdot Na_2$ |
|---|---|---|
| | $R = C_4H_9$—, Z = -NH-, f = 1 | |
| *HCA[c](1) | 1.0:1.0 | |
| 1 | 20.0:1.0 | 79:21 |
| 2 | 10.0:1.0 | 77:23 |
| 3 | 6.7:1.0 | 82:18 |
| | $R = C_4H_9$—, Z = -O-, f = 1 | |
| *HCA(2) | 1.0:1.0 | |
| 4 | 20.0:1.0 | 82:18 |
| 5 | 10.0:1.0 | 84:16 |
| 6 | 6.7:1.0 | 85:15 |

TABLE I (cont'd)

| Sample | Mole Ratio ESA · $Na_2$ : R - [-Z-H]$_f$ | Composition[b] Wt. % Actives: Wt. % TA· $Na_2$ |
|---|---|---|
| | $R = C_6H_{13}$—, Z = -NH-, f = 1 | |
| ∝HCA(3) | 1.0:1.0 | |
| 7 | 20.0:1.0 | 72:28 |
| 8 | 10.0:1.0 | 80:20 |
| 9 | 6.7:1.0 | 78:22 |
| | $R = C_6H_5-CH_2-$, Z = -NH-, f = 1 | |
| ∝HCA(4) | 1.0:1.0 | |
| 10 | 20.0:1.0 | 81:19 |
| 11 | 10.0:1.0 | 84:16 |
| | $R = C_6H_5-CH_2-$, Z = -NH-, f = 1 | |
| ∝HCA(5) | 1.0:1.0 | |
| 12 | 10.0:1.0 | 83:17 |
| | $R = (HOCH_2CH_2)_2$—, Z = N-, f = 1 | |
| ∝HCA(6) | 1.0:1.0 | |
| 13 | 20.0:1.0 | 80:20 |
| 14 | 10.0:1.0 | 81:19 |
| 15 | 6.7:1.0 | 84:16 |

TABLE I (cont'd)

| Sample | Mole Ratio ESA · $Na_2$ : R - [-Z-H]$_f$ | Composition[b] Wt. % Actives: Wt. % TA· $Na_2$ |
|---|---|---|
| | R = $(HOCH_2)_3C$—, Z = -NH-, f = 1 | |
| ∝HCA(7) | 1.0:1.0 | |
| 16 | 10.0:1.0 | 81:19 |
| | Z = $NaO_3S$— | |
| ∝HCA(8) | 1.0:1.0 | |
| 17 | 20.0:1.0 | 86:14 |
| 18 | 10.0:1.0 | 86:14 |
| 19 | 10.0:1.0 | 81:19 |
| | R = $NaO_3S$-$CH_2CH_2$—, Z = -NH-, f = 1 | |
| ∝HCA(9) | 1.0:1.0 | |
| 20 | 20.0:1.0 | 76:24 |
| 21 | 10.0:1.0 | 79:21 |
| 22 | 6.7:1.0 | 83:17 |
| 23 | 20.0:1.0 | 78:22 |
| 24 | 10.0:1.0 | 81:19 |
| 25 | 6.7:1.0 | 84:16 |

TABLE I (cont'd)

| Sample | Mole Ratio ESA · $Na_2$ : R - [-Z-H]$_f$ | Composition[b] Wt. % Actives: Wt. % TA· $Na_2$ |
|---|---|---|
| | R = $HOCH_2(CHOH)_3C(CO_2H)$—, Z = -O-, f = 1 | |
| 26 | 20.0:1.0 | 78:22 |
| 27 | 10.0:1.0 | 80:20 |
| 28 | 6.7:1.0 | 83:17 |
| | R = $-C(CO_2H)(CHOH)_2C(CO_2H)$—, Z = -O-, f = 2 | |
| 29 | 20.0:1.0 | 78:22 |
| 30 | 10.0:1.0 | 81:19 |
| 31 | 6.7:1.0 | 85:15 |
| | R = $-(C_6H_{12})$—, Z = -NH-, f = 2 | |
| ~HCA(10) | 1.0:1.0 | |
| 32 | 20.0:1.0 | 80:20 |
| 33 | 10.0:1.0 | 83:17 |
| 34 | 6.7:1.0 | 85:15 |

TABLE I (cont'd)

| Sample | Mole Ratio ESA · $Na_2$ : R - [-Z-H]$_f$ | Composition[b] Wt. % Actives: Wt. % TA· $Na_2$ |
|---|---|---|
| R = meta $-CH_2C_6H_4-CH_2-$, Z = -NH-, f = 2 | | |
| ∝HCA(11) | 1.0:1.0 | |
| 35 | 20.0:1.0 | 82:18 |
| 36 | 10.0:1.0 | 83:17 |
| 37 | 6.7:1.0 | 85:15 |
| R = para $-CH_2-C_6H_4-CH_2-$, Z = -NH-, f = 2 | | |
| ∝HCA(12) | 1.0:1.0 | |
| 38 | 20.0:1.0 | 79:21 |
| 39 | 10.0:1.0 | 84:16 |
| 40 | 6.7:1.0 | 85:15 |
| R = para $-CH_2-C_6H_4-CH_2-$, Z = -S-, f = 2 | | |
| ∝HCA(13) | 1.0:1.0 | |
| 41 | 6.7:1.0 | 87:13 |

[a] Mole ratio of $Ca(OH)_2$ : ESA · $Na_2$ + ∝HCA (Scheme A) or ESA · $Na_2$ (Scheme B) was 1:10 for all reactions.

[b] Reported as a weight percent of the organic solid content of the product; TA · $Na_2$ stands for disodium tartrate.

[c] Corresponding ∝HCA analog, n = 1

Example IV

Table II summarizes the static calcium carbonate inhibition testing of the compounds of the present invention compared to several prior art calcium carbonate control agents. The tests were performed by adding the treatment solution (sample) to a carbonate stock solution of the described conditions.

A calcium stock solution was then added and the mixture was incubated for 17 hours at 70°C. All treatments were adjusted to pH 9.0 prior to use and the treatment weights, expressed as the sodium salts, accounted for the presence of disodium tartrate by-product. After cooling, a measured portion of the mixture was filtered and the filtrate pH adjusted to less than 1 with hydrochloric acid. The filtrate was then diluted and pH adjusted to 12 with sodium hydroxide. A calcium indicator, murexide, was then added and the solution titrated with a known concentration of ethylenediaminetetraacetic acid (EDTA). From titrations for the treated, stock and control solutions, the percent inhibition was calculated as follows:

$$\% \text{ Inhibition} = \frac{\text{mls EDTA(treated) - mls EDTA(control)}}{\text{mls EDTA(stock) - mls EDTA(control)}} \times 100$$

Typically, the test samples were evaluated twice referenced to a sample of PESA. The compounds of the present invention were in general as effective as PESA, and more effective than HEDP and PBSAM at 10 ppm actives regardless of the mole % of αHCA compound used in the synthesis. At 5 ppm actives a decrease in efficacy was observed with increasing αHCA content; however, at lower levels of αHCA the activity of the compounds of the present invention generally exceeded PESA and were equivalent to that of PBSAM.

The αHCA compounds alone did not exhibit any calcium carbonate inhibition activity.

TABLE II

| Static Calcium Carbonate % Inhibition Evaluation | | |
|---|---|---|
| Conditions: | 1106 ppm Ca as $CaCO_3$<br>1160 ppm $CO_3$ as $CaCO_3$<br>538 ppm Na<br>784 ppm Cl<br>518 ppm $SO_4$ | Stock Solutions pH = 9.0<br>Temp. = 70°C<br>Duration = 17 hours |
| Sample | 5 ppm Actives | 10 ppm Actives |
| αHCA(1) | 2.3 | 0.7 |
| 1 | 81.8 | 96.9 |
| 2 | 82.9 | 97.1 |
| 3 | 60.5 | 95.1 |
| αHCA(2) | 1.4 | 4.4 |
| 4 | 67.9 | 85.7 |
| 5 | 70.5 | 85.2 |
| 6 | 61.8 | 83.5 |
| αHCA(3) | 2.4 | 2.2 |
| 7 | 74.6 | 87.8 |
| 8 | 75.2 | 89.1 |
| 9 | 59.6 | 92.0 |
| αHCA(4) | 0.0 | 0.0 |
| 10 | 75.5 | 87.8 |
| 11 | 70.1 | 87.7 |
| 13 | 55.0 | 65.5 |
| 14 | 60.9 | 72.4 |
| 16 | 58.6 | 76.1 |
| αHCA(8) | 4.4 | |
| 17 | 56.6 | 86.3 |
| 18 | 60.0 | 85.4 |
| 19 | 74.3 | 76.8 |
| αHCA(9) | 5.1 | 3.7 |
| 20 | 77.1 | 91.3 |
| 21 | 61.5 | 89.8 |

TABLE II (continued)

| Static Calcium Carbonate % Inhibition Evaluation | | |
|---|---|---|
| Sample | 5 ppm Actives | 10 ppm Actives |
| 22 | 44.8 | 83.0 |
| 23 | 79.6 | 96.5 |
| 24 | 58.9 | 93.2 |
| 25 | 46.6 | 83.6 |
| 26 | 66.5 | 80.6 |
| 27 | 52.7 | 80.4 |
| 28 | 43.2 | 78.8 |
| 29 | 70.1 | 82.8 |
| 30 | 59.0 | 77.9 |
| 31 | 44.3 | 77.9 |
| αHCA(10) | 6.7 | 3.3 |
| 32 | 79.2 | 91.6 |
| 33 | 75.8 | 95.4 |
| 34 | 62.0 | 92.0 |
| αHCA(11) | 0.0 | 8.3 |
| 35 | 75.9 | 91.3 |
| 36 | 80.7 | 96.5 |
| 37 | 81.0 | 98.2 |
| αHCA(12) | 1.3 | 0.9 |
| 38 | 77.7 | 91.1 |
| 39 | 80.5 | 93.0 |
| 40 | 76.0 | 96.5 |
| PESA[1] | 58.6 | 90.6 |
| PBSAM[2] | 67.5 | 76.0 |
| HEDP[3] | 73.1 | 71.3 |
| K-752[4] | 78.8 | 84.0 |
| K-732[5] | 68.4 | 74.6 |
| SCP-1[6] | 42.1 | 45.5 |

[1] PESA = Poly[2,3-oxiranedicarboxylic acid], refer to Brown et al., U.S. Patent 5,062,962. Average of 12 tests.
[2] PBSAM = 2-phosphonobutane - 1,2,4-tricarboxylic acid (Mobay Chemical Co.). Average of 3 tests.
[3] HEDP = 1-hydroxyethylidene-1,1-diphosphonic acid (Monsanto Co.). Average of 5 tests.
[4] K-752 = Goodrite K-752, poly[acrylic acid] (B.F. Goodrich Co.)
[5] K-732 = Goodrite K-732, poly[acrylic acid] (B.F. Goodrich Co.)
[6] SCP-1 = Sulfonated copolymer; refer to Chen, U.S. Patent 4,654,159

The corrosion inhibition activity of the present invention was evaluated with a Beaker Corrosion Test Apparatus (BCTA). The BCTA is composed of a 2 liter beaker equipped with an air/$CO_2$ sparge, 1010 LCS coupon, 1010 LCS electrochemical probe and magnetic stirrer. The test solution volume is 1.9 liters.

Electrochemical corrosion rate data (EC) are obtained during the test from potentiodynamic polarization resistance measurements. Additional corrosion data is obtained from the coupon and the electrochemical probe by standard weight loss measurement techniques. All tests were conducted under the following conditions unless otherwise noted:

| | |
|---|---|
| 250 mg/l Ca as $CaCO_3$ | pH 8.4 |
| 125 mg/l Mg as $CaCO_3$ | Temperature 120°F |
| 10 mg/l $SiO_2$ | 600 ppmv $CO_2$ Air Sparge |
| 300 mg/l Chloride | 400 rpm stirring |
| 200 mg/l Sulfate | 40 hours duration |
| 134 mg/l $NaHCO_3$ | Nominal M alk 90 mg/l as $CaCO_3$ |

Under these conditions, the test water is supersaturated with respect to calcium carbonate. It is known in the art that a precipitated film of calcium carbonate will inhibit corrosion, resulting in much lower corrosion rates than that resulting from the inhibitors themselves. Therefore, all tests were conducted with a base treatment to inhibit the precipitation of calcium carbonate under the test conditions. The blank runs reported for all tests include the addition of the base treatment.

It was observed during the course of the testing that, on average, the test coupons were more sensitive to the lack of an effective inhibitor than was the test probe. Consequently, tests of inhibitors with slight to moderate effectiveness exhibited higher corrosion rates for the coupon weight loss data than for rates obtained for the electrochemical data or test probe weight loss data. Such compounds are not judged to be ineffective, but rather are less effective than compounds which produced consistent and lower corrosion rates for coupon, probe and electrochemical test data.

In all tests, the blank corrosion rates are expressed in mils per year (mpy). The corrosion rates for the inhibitor compounds are expressed as a reduction in the corrosion rate relative to the blank calculated according to the following equation:

$$\text{\% Corrosion Inhibition} = \frac{(\text{mpy Blank - mpy Treated})}{\text{mpy Blank}} \times 100$$

For the purpose of this invention, a particularly effective corrosion inhibitor will reduce the coupon weight loss corrosion rate by at least 80% compared to the blank. All tests were conducted as parts per million of the inhibitor molecule as the sodium salt. Compound αHCA(12) was evaluated as ppm of the free acid.

Table III illustrates the percent corrosion inhibition results for the compounds of the present invention, and some of their corresponding αHCA analogs. The base treatment is made up of a combination of 1 ppm of 1-hydroxyethane-1,1-diphosphonic acid (HEDP), 10 ppm acrylic copolymer (see Chen, U.S. Patent 4,659,481) and 15 ppm molybdate as $MoO_4^{2-}$. The molybdate content of the base treatment is not sufficient to provide adequate corrosion protection, as demonstrated by the high corrosion rate for the blank. A sample of poly[epoxysuccinic acid] (PESA) of similar molecular weight as the compounds of the present invention was also evaluated and found to provide inadequate corrosion protection.

These results demonstrate that, in general, the monofunctional derivatives of PESA (Formula I, f = 1; n > 1 ) are significantly more effective than the corresponding αHCA analogs. The difunctional derivatives of PESA (Formula I, f = 2; n > 1) were comparable to the corresponding αHCA analogs.

TABLE III

| Percent Corrosion Inhibition Modified PESA Compounds with Molybdate | | | |
|---|---|---|---|
| Example (25 ppm) | Coupon | Probe | EC (avg) |
| Blank | 62.4 mpy | 58.9 mpy | 47.1 mpy |
| PESA[1] | 37.3 | -10.5 | 23.6 |
| αHCA(1) | -22.6 | 52.5 | 63.3 |
| 1 | 69.7 | 93.2 | 93.8 |
| αHCA(2) | 46.3 | 88.1 | 73.5 |
| 5 | 73.6 | 86.1 | 90.0 |

[1] No HEDP was added with the blank.

TABLE III (continued)

| Percent Corrosion Inhibition Modified PESA Compounds with Molybdate | | | |
|---|---|---|---|
| Example (25 ppm) | Coupon | Probe | EC (avg) |
| αHCA(3) | -29.4 | 42.6 | 47.6 |
| 7 | 63.6 | 81.7 | 83.4 |
| αHCA(4) | 44.9 | -[2] | 7.4 |
| 10 | 83.5 | -[2] | 63.7 |
| αHCA(5) | 14.3 | 27.7 | 32.3 |
| 12 | 89.1 | 82.3 | 86.8 |
| αHCA(6) | 76.0 | 86.6 | 86.6 |
| 15 | 74.0 | 96.4 | 87.9 |
| αHCA(7) | 56.4 | 85.2 | 83.7 |
| 16 | 86.9 | 84.4 | 87.9 |
| 18 | 84.0 | 80.3 | 82.2 |
| αHCA(9) | 29.3 | 66.2 | 69.4 |
| 20 | 73.1 | 91.2 | 88.1 |
| 28 | 91.0 | 89.6 | 90.5 |
| 30 | 68.1 | 87.1 | 88.8 |
| αHCA(10) | 84.0 | 92.9 | 93.8 |
| 32 | 71.3 | 89.5 | 92.6 |
| αHCA(11) | 88.0 | 88.6 | 85.1 |
| 37 | 85.7 | 87.1 | 91.1 |
| αHCA(12) | 91.5 | 93.4 | 96.0 |
| 39 | 92.6 | 95.9 | 96.4 |
| αHCA(13) | 49.8 | 84.6 | 87.1 |
| 41 | 60.0 | 86.4 | 83.3 |

[2] Data not included because probe was not polished prior to use.

## Claims

**1.** A compound having the formula:

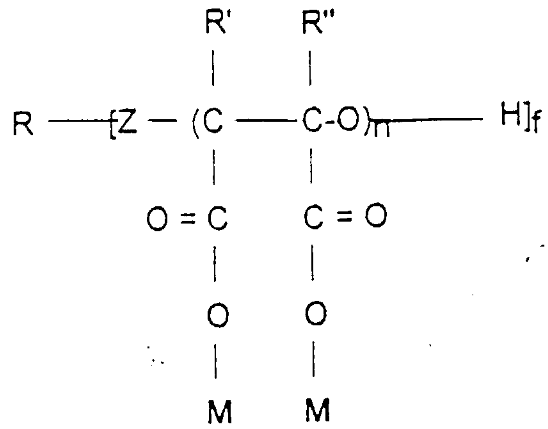

wherein R is alkyl or substituted alkyl, or aryl or substituted aryl; R' and R'' are each independently hydrogen, $C_{1-4}$ alkyl or $C_{1-4}$ substituted alkyl; Z is NH, NR, O or S; n is a positive integer greater than 1; f is a positive integer; and each M independently is H, a water soluble cation or a $C_{1-3}$ alkyl group.

**2.** A compound as claimed in claim 1, wherein R is $C_1$-$C_{20}$ alkyl or $C_1$-$C_{20}$ substituted alkyl.

**3.** A compound as claimed in claim 1, wherein R is aryl or substituted aryl containing 4-9 carbon atoms.

**4.** A compound as claimed in claim 1, wherein R is aryl containing 4-6 carbon atoms.

**5.** A compound as claimed in claim 1, wherein R is $-CH_2C_6H_4CH_2-$ and f is 2.

**6.** A compound as claimed in claim 1, wherein R is $HOCH_2(CHOH)_3C(CO_2H)-$ and f is 1.

**7.** A compound as claimed in any one of the preceding claims, wherein M is $Na^+$.

**8.** A scale and/or corrosion inhibitor composition useful for treating an aqueous system comprising a combination of:

(a) a compound as claimed in any one of the preceding claims, and
(b) a topping agent selected from the group consisting of polyacrylates, phosphoric acids and water soluble salts thereof, phosphonic acids and water soluble salts thereof, polyvalent metal salts and azole compounds.

**9.** A method for controlling the formation and deposition of scale forming salts in an aqueous system comprising introducing into said aqueous system a sufficient amount for the purpose of a treatment comprising a compound as claimed in any one of claims 1 to 7.

**10.** A method as claimed in claim 9,wherein said compound is added to the aqueous system at active treatment levels ranging from 25 parts per billion to 500 parts per million.

**11.** A method as claimed in claim 10, wherein said compound is added to the aqueous system at active treatment levels ranging from 50 parts per billion to 100 parts per million.

**12.** A method as claimed in claim 9, 10 or 11, wherein said scale forming salts include calcium carbonate.

**13.** A method for controlling the corrosion of metals in contact with an aqueous system comprising introducing into said aqueous system a sufficient amount for the purpose of a treatment comprising a compound as claimed in any one of claims 1 to 7.

**14.** A method as claimed in claim 13, wherein said compound is added to the aqueous system at active treatment levels ranging from 0.025 to 500 parts per million.

**15.** A method as claimed in claim 14, wherein said compound is added to the aqueous system at active treatment levels ranging from 0.05 to 100 parts per million.

**16.** A method as claimed in any one of claims 13 to 15, wherein said metals are ferrous-based.

**17.** A method as claimed in any one of claims 9 to 16, further comprising adding to said aqueous system a sufficient amount for the purpose of a topping agent selected from the group consisting of polyacrylates, phosphoric acids and water soluble salts thereof, phosphonic acids and water soluble salts thereof, polyvalent metal salts and azole compounds.

**18.** A method as claimed in claim 17, wherein said polyacrylate has the formula:

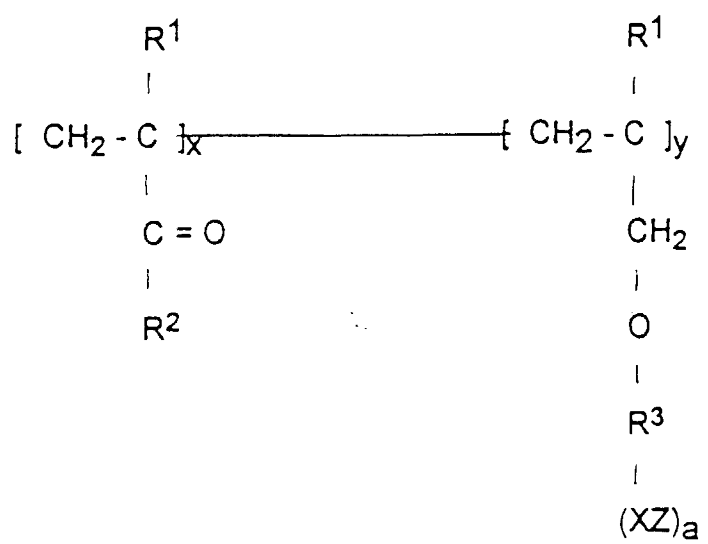

wherein each $R^1$ is independently H or lower alkyl; $R^2$ is OH, $NH_2$ or OM: M is a water soluble cation; $R^3$ is a hydroxy substituted alkyl or alkylene radical having from 1 to 6 carbon atoms; x is $SO_3$, $PO_3$, $PO_4$ or $CO_2$; Z is H or a water soluble cation or cations; and a is 0 or 1.

**19.** The method as claimed in claim 18, wherein the molar ratio of x:y is from 30:1 to 1:20.

**20.** A method as claimed in any one of claims 9 to 19, wherein said aqueous system is a cooling water system.

**21.** A method as claimed in any one of claims 9 to 19, wherein said aqueous system is a steam generating system.

**22.** A method as claimed in any one of claims 9 to 19, wherein said aqueous system is a gas scrubbing system.

**23.** A method as claimed in any one of claims 9 to 19, wherein said aqueous system is a pulp and papermaking system.

## Patentansprüche

**1.** Verbindung, welche die folgende Formel aufweist:

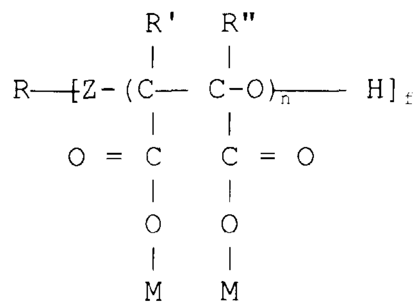

worin R Alkyl oder substituiertes Alkyl oder Aryl oder substituiertes Aryl ist; R' und R" jeweils unabhängig Wasserstoff, $C_{1-4}$-Alkyl oder substituiertes $C_{1-4}$-Alkyl sind; Z NH, NR, O oder S ist; n eine positive ganze Zahl größer als 1 ist; f eine positive ganze Zahl ist und jedes M unabhängig H, ein wasserlösliches Kation oder eine $C_{1-3}$-Alkylgruppe ist.

**2.** Verbindung nach Anspruch 1, worin R $C_{1-20}$-Alkyl oder substituiertes $C_{1-20}$-Alkyl ist.

**3.** Verbindung nach Anspruch 1, worin R Aryl oder substituiertes Aryl ist, das 4 - 9 Kohlenstoffatome enthält.

**4.** Verbindung nach Anspruch 1 worin R Aryl ist, das 4 - 6 Kohlenstoffatome enthält.

**5.** Verbindung nach Anspruch 1, worin R $-CH_2C_6H_4CH_2-$ ist und f 2 ist.

**6.** Verbindung nach Anspruch 1, worin R
$HOCH_2(CHOH)_3C(CO_2H)-$ ist und f 1 ist.

**7.** Verfahren nach einem der vorangehenden Ansprüche, worin M $Na^+$ ist.

**8.** Kesselstein- und/oder Korrosionsinhibitor-Zusammensetzung, die zur Behandlung eines wäßrigen Systems nützlich ist, die eine Kombination aus folgendem umfaßt:

(a) Eine Verbindung nach einem der vorangehenden Ansprüche und

(b) ein Toppingmittel, das aus der Gruppe ausgewählt wird, die aus Polyacrylaten, Phosphorsäuren und wasserlöslichen Salzen davon, Phosphonsäuren und wasserlöslichen Salzen davon, polyvalenten Metallsalzen und Azolverbindungen besteht.

**9.** Verfahren zur Kontrolle der Bildung und Ablagerung von kesselsteinbildenden Salzen in einem wäßrigen System, welches das Einführen in genanntes wäßriges System einer ausreichenden Menge für den Zweck einer Behandlung umfaßt, die eine Verbindung nach einem der Ansprüche 1 bis 7 umfaßt.

**10.** Verfahren nach Anspruch 9, worin genannte Verbindung dem wäßrigen System bei aktiven Behandlungskonzentrationen zugesetzt wird, die von 25 Teilen auf eine Milliarde (ppb) bis 500 Teile auf eine Million (ppm) reichen.

**11.** Verfahren nach Anspruch 10, worin genannte Verbindung dem wäßrigen System bei aktiven Behandlungskonzentrationen zugesetzt wird, die von 50 Teilen auf eine Milliarde (ppb) bis 100 Teile auf 1 Million (ppm) reichen.

**12.** Verfahren nach Anspruch 9, 10 oder 11, worin genannte kesselsteinbildenden Salze Calciumcarbonat einschließen.

**13.** Verfahren zur Kontrolle der Korrosion von Metallen in Kontakt mit einem wäßrigen System, das die Einführung in genanntes wäßriges System einer ausreichenden Menge für den Zweck einer Behandlung umfaßt, die eine Verbindung nach einem der Ansprüche 1 bis 7 umfaßt.

**14.** Verfahren nach Anspruch 13, worin genannte Verbindung dem wäßrigen System bei aktiven Behandlungskonzentrationen zugesetzt wird, die von 0,025 Teilen bis 500 Teile auf 1 Million (ppm) reichen.

**15.** Verfahren nach Anspruch 14, worin genannte Verbindung dem wäßrigen System bei aktiven Behandlungskonzentrationen zugesetzt wird, die von 0,05 Teilen bis 100 Teile pro 1 Million (ppm) reichen.

**16.** Verfahren nach einem der Ansprüche 13 bis 15, worin genannte Metalle auf Eisen(II)-basiert sind.

**17.** Verfahren nach einem der Ansprüche 9 bis 16, das überdies das Zusetzen zu genanntem wäßrigem System einer ausreichenden Menge für den Zweck eines Toppingmittels umfaßt, das aus der Gruppe ausgewählt wird, die aus Polyacrylaten, Phosphorsäuren und wasserlöslichen Salzen davon, Phosphonsäuren und wasserlöslichen Salzen davon, polyvalenten Metallsalzen und Azolverbindungen besteht.

**18.** Verfahren nach Anspruch 17, worin genanntes Polyacrylat die folgende Formel aufweist:

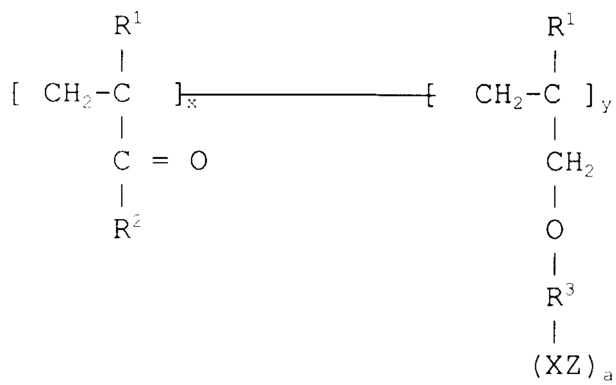

worin jedes $R^1$ unabhängig H oder ein niederes Alkyl ist; $R^2$ OH, $NH_2$ oder OM (M ein wasserlösliches Kation ist) ist; $R^3$ ein Hydroxy-substituiertes Alkyl oder Alkylenradikal mit von 1 bis 6 Kohlenstoffatomen ist; x $SO_3$, $PO_3$, $PO_4$ oder $CO_2$ ist; Z H oder ein wasserlösliches Kation oder wasserlösliche Kationen ist und a 0 oder 1 ist.

**19.** Verfahren nach Anspruch 18, worin sich das Molverhältnis von x:y von 30:1 bis 1:20 bewegt.

**20.** Verfahren nach einem der Ansprüche 9 bis 19, worin genanntes wäßriges System ein Kühlwassersystem ist.

**21.** Verfahren nach einem der Ansprüche 9 bis 19, worin genanntes wäßriges System ein dampferzeugendes System ist.

**22.** Verfahren nach einem der Ansprüche 9 bis 19, worin genanntes wäßriges System ein Gaswaschsystem ist.

**23.** Verfahren nach einem der Ansprüche 9 bis 19, worin genanntes wäßriges System ein Zellstoff- und Papierherstellungssystem ist.

## Revendications

**1.** Composé ayant la formule:

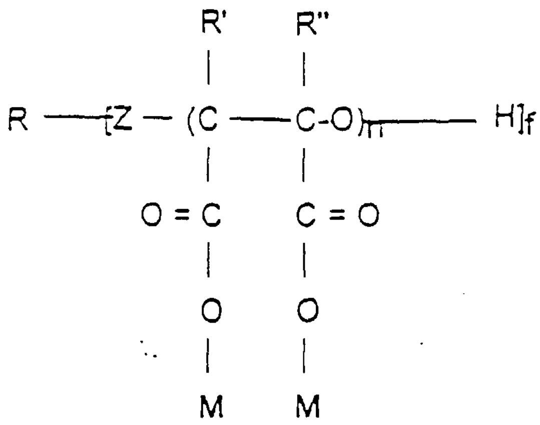

où R est un alkyle, un alkyle substitué, ou un aryle ou un aryle substitué; R' et R" sont chacun indépendamment de l'hydrogène, un alkyle $C_{1-4}$ ou un alkyle $C_{1-4}$ substitué; Z est NH, NR, O ou S; n est un nombre entier positif supérieur à 1; f est un nombre entier positif; et chaque M indépendamment est H, un cation soluble dans l'eau ou un groupe alkyle $C_{1-3}$.

**2.** Composé tel que revendiqué dans la revendication 1, dans lequel R est un alkyle $C_1$-$C_{20}$ ou un alkyle $C_1$-$C_{20}$ substitué.

**3.** Composé tel que revendiqué dans la revendication 1, dans lequel R est un aryle ou un aryle substitué contenant 4-9 atomes de carbone.

**4.** Composé tel que revendiqué dans la revendication 1, dans lequel R est un aryle contenant 4-6 atomes de carbone.

**5.** Composé tel que revendiqué dans la revendication 1, dans lequel R est $-CH_2C_6H_4CH_2-$ et f est 2.

**6.** Composé tel que revendiqué dans la revendication 1, dans lequel R est $HOCH_2(CHOH)_3C(CO_2H)-$ et f est 1.

**7.** Composé tel que revendiqué dans l'une quelconque des revendications précédentes, dans lequel M est $Na^+$.

**8.** Composition inhibitrice de tartre et/ou de corrosion utile pour le traitement d'un système aqueux comprenant une combinaison de:

(a) un composé tel que revendiqué dans l'une quelconque des revendications précédentes, et
(b) un agent de fractionnement sélectionné à partir du groupe consistant en polyacrylates, en acides phosphoriques et sels de ceux-ci solubles dans l'eau, en acides phosphoniques et sels de ceux-ci solubles dans l'eau, en sels métalliques polyvalents et en composés pyrroliques.

**9.** Procédé pour contrôler la formation et le dépôt de sels formant tartre dans un système aqueux comprenant introduire dans ledit système aqueux une quantité suffisante dans le but, d'un traitement comprenant un composé tel que revendiqué dans l'une quelconque des revendications 1 à 7.

**10.** Procédé tel que revendiqué dans la revendication 9, dans lequel ledit composé est ajouté au système aqueux à des niveaux de traitement actif allant de 25 parties par milliard à 500 parties par million.

**11.** Procédé tel que revendiqué dans la revendication 10, dans lequel ledit composé est ajouté au système aqueux à des niveaux de traitement actif allant de 50 parties par milliard à 100 parties par million.

**12.** Procédé tel que revendiqué dans la revendication 9, 10 ou 11, dans lequel les sels formant tartre incluent du carbonate de calcium.

**13.** Procédé pour contrôler la corrosion des métaux en contact avec un système aqueux comprenant introduire dans ledit système aqueux une quantité suffisante dans le but, d'un traitement comprenant un composé tel que revendiqué dans l'une quelconque des revendications 1 à 7.

**14.** Procédé tel que revendiqué dans la revendication 13, dans lequel ledit composé est ajouté au système aqueux à des niveaux de traitement actif allant de 0,025 à 500 parties par million.

**15.** Procédé tel que revendiqué dans la revendication 14, dans lequel ledit composé est ajouté au système aqueux à des niveaux de traitement actif allant de 0,05 à 100 parties par million.

**16.** Procédé tel que revendiqué dans l'une quelconque des revendications 13 à 15, dans lequel lesdits métaux sont à base ferreuse.

**17.** Procédé tel que revendiqué dans l'une quelconque des revendications 9 à 16, comprenant en outre ajouter audit système aqueux une quantité suffisante dans le but, d'un agent de fractionnement sélectionné à partir du groupe consistant en polyacrylates, en acides phosphoriques et en sels de ceux-ci solubles dans l'eau, en acides phosphoniques et en sels de ceux-ci solubles dans l'eau, en sels métalliques polyvalents et en composés pyrroliques.

**18.** Procédé tel que revendiqué dans la revendication 17, dans lequel ledit polyacrylate a la formule:

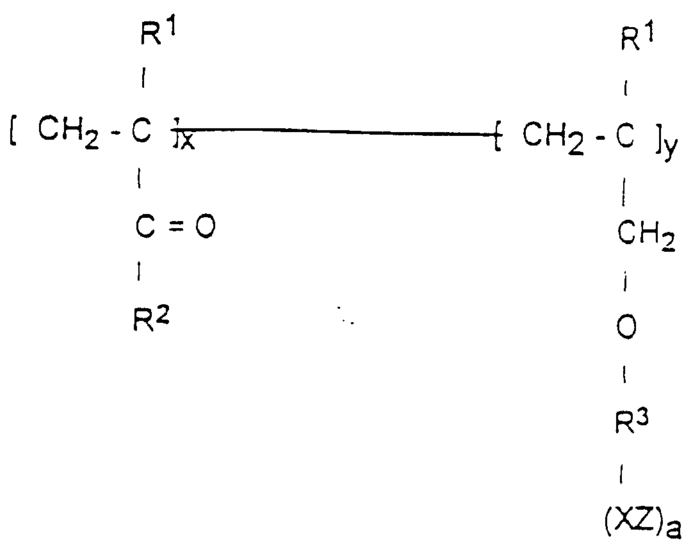

où chaque $R^1$ est indépendamment H ou un alkyle inférieur; $R^2$ est OH, $NH_2$ ou OM: M est un cation soluble dans l'eau; $R^3$ est un alkyle substitué hydroxy ou un radical alkylène ayant de 1 à 6 atomes de carbone; x est $SO_3$, $PO_3$, $PO_4$ ou $CO_2$; Z est H ou un cation ou des cations solubles dans l'eau; et a est O ou 1.

**19.** Procédé tel que revendiqué dans la revendication 18, dans lequel le rapport molaire de x:y est de 30:1 à 1:20.

**20.** Procédé tel que revendiqué dans l'une quelconque des revendications 9 à 19, dans lequel ledit système aqueux est un système à eau de refroidissement.

**21.** Procédé tel que revendiqué dans l'une quelconque des revendications 9 à 19, dans lequel ledit système aqueux est un système générateur de vapeur.

**22.** Procédé tel que revendiqué dans l'une quelconque des revendications 9 à 19, dans lequel ledit système aqueux est un système d'épuration de gaz.

**23.** Procédé tel que revendiqué dans l'une quelconque des revendications 9 à 19, dans lequel ledit système aqueux est un système de fabrication de pâte et de papier.